(19) Europäisches Patentamt
European Patent Office
Office européen des brevets

(11) Veröffentlichungsnummer: **0 078 414**
**B1**

(12) # EUROPÄISCHE PATENTSCHRIFT

(45) Veröffentlichungstag der Patentschrift: **19.12.84**

(51) Int. Cl.$^3$: **C 07 C 85/24**, C 07 C 87/50

(21) Anmeldenummer: **82109328.3**

(22) Anmeldetag: **08.10.82**

(54) **Verfahren zur Herstellung von Diaminen der Diphenylmethanreihe.**

(30) Priorität: **27.10.81 DE 3142529**

(43) Veröffentlichungstag der Anmeldung: **11.05.83 Patentblatt 83/19**

(45) Bekanntmachung des Hinweises auf die Patenterteilung: **19.12.84 Patentblatt 84/51**

(84) Benannte Vertragsstaaten: **AT BE CH DE FR GB IT LI LU NL SE**

(56) Entgegenhaltungen:
**EP-A- 0 000 778**
**EP-A- 0 009 094**
**EP-A- 0 014 927**
**FR-A- 1 377 734**
**US-A- 3 882 093**
**US-A- 4 041 078**

(73) Patentinhaber: **CHEMIE LINZ AKTIENGESELLSCHAFT**
**St. Peter-Strasse 25**
**A-4020 Linz (AT)**
**BE CH FR GB IT LI LU NL SE AT**
**Lentia Gesellschaft mit beschränkter Haftung**
**Arabellastrasse 4 Postfach 81 05 08**
**D-8000 München 81 (DE)**
**DE**

(72) Erfinder: **Salschek, Gerald, Dipl. Ing.**
**Roseggerstrasse 4**
**A-4600 Wels (AT)**
Erfinder: **Fuchs, Franz**
**Schumpeterstrasse 2**
**A-4045 Linz (AT)**
Erfinder: **Stern, Gerhard, Dipl. Ing. Dr.**
**Lustenauerstrasse 17**
**A-4020 Linz (AT)**

**EP 0 078 414 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist die Herstellung von Diaminen der Diphenylmethanreihe durch Umsetzung von Arylaminen mit gegenüber der zur Aminogruppe freien para-Stellung mittels Formaldehyd mit einem sauren Ionentauscher als Katalysator, wobei der vielfach unerwünscht hohe Anteil an mehrkernigen Kondensationsprodukten im Gegensatz zu allen bisher bekannten Verfahren im Durchschnitt 5 % nicht überschreitet.

Bekanntlich erfolgt die Bildung von Diaminen der Diphenylmethanreihe aus den entsprechenden Aminen und Formaldehyd über die Kondensation zu Methylendianilinen bzw. Aminobenzylanilinen, die dann mit Hilfe von sauren Katalysatoren in die Endprodukte umgelagert werden, wobei der in der europäischen Patentanmeldung 0 014 927 angegebene Reaktionsablauf gelten dürfte.

In der DE-AS 1 210 872 wurde bereits vorgeschlagen, als saure Katalysatoren Säuren zu verwenden, die während der Umsetzung in heterogener Phase vorliegen, wobei als Ionentauscher bekannte, sulfonierte Copolymerisate von Styrol und Divinylbenzol speziell hervorgehoben wurden. Dadurch konnte im Vergleich zu den früher üblichen Verfahren mit Mineralsäuren als Katalysatoren die aufwendige Aufarbeitung vermieden werden.

Derartige feste, stark saure Harze wurden neben schwächer sauren Ionentauschern auch gemäß DE-OS 20 37 550 als Katalysator für die Umsetzung von Arylaminen mit Formaldehyd eingesetzt, wobei hervorgehoben wird, daß innerhalb der Fraktion von zweikernigen Verbindungen die Bildung an 2,4'-Isomeren der Dimethylamino-diphenylmethane durch Erhöhung der Temperatur während der Umlagerung sowie durch Erhöhung des Molverhältnises von Amin zu Formaldehyd begünstigt wird, während gleichzeitig die Menge an gebildetem 4,4'-Isomeren nachläßt. Der Gehalt an Aminen mit 3 und mehr Kernen im Polymergemisch liegt dabei meist um 20 bis 35 Gew.% und nur in einem Fall bei einstufiger Umsetzung bei 190 °C unter 10 %, nämlich bei 7,8 %, wobei über die Ausbeute an Di- bzw. Polyaminen und deren Reinheit nichts ausgesagt ist. Während der Umlagerung wird bei diesem Verfahren das Kondensationswasser laufend entfernt.

Hingegen wird gemäß EU-OS 778 das Abdestillieren des Kondensationswassers bei der gleichen Reaktion und bei Verwendung von sulfonierten Styrol-Divinylbenzol-Copolymerisaten eines bestimmten Vernetzungsgrades unterbunden und damit ein hoher Gehalt des 4,4'-Isomeren in der Diaminfraktion erzielt. Der Gehalt an 3- bzw. 4-Kernverbindungen beträgt bei diesem Verfahren stets deutlich über 10 %, nämlich 14 bis 18 %.

Schließlich wurde gemäß US-PS 4 041 078 schon vorgeschlagen, fluorhältige Katalysatoren, nämlich fluorierten Graphit, einzusetzen. Der Gehalt an Diaminen beträgt bei diesem Verfahren aber sogar nur 43,8 bis 64,6 %.

Da jedoch vor allem die Zweikernverbindungen wertvolle Ausgangsprodukte für die Herstellung von Diisocyanaten darstellen, ist ein hoher Anteil an Polyaminen unerwünscht.

Überraschenderweise wurde nun gefunden, daß der Gehalt des Endproduktes an mehrkernigen Verbindungen auf unter 5 % gesenkt werden kann, wenn man als Katalysator Perfluoralkylgruppen und Sulfonsäuregruppen enthaltende Ionenaustauscher einsetzt. Die dabei erhaltenen Diamingemische zeichnen sich darüberhinaus dadurch aus, daß sie praktisch keine Verunreinigungen an nicht umgelagerten Vorprodukten enthalten.

Gegenstand der Erfindung ist somit ein Verfahren zur Herstellung von Diaminen der Diphenylmethanreihe durch Kondensation von einkernigen Arylaminen mit freier para-Stellung gegenüber der Aminogruppe mit Formaldehyd oder unter Reaktionsbedingungen Formaldehyd abgebenden Substanzen und anschließende Umlagerung bei erhöhter Temperatur und in Gegenwart eines sauren, Sulfonsäuregruppen enthaltenden Ionentauschers als Katalysator, wobei in beiden Stufen Wasser zugegen ist, dadurch gekennzeichnet, daß als Katalysator, der zumindest bei der Umlagerung zugegen ist, ein Copolymeres von Tetrafluoräthylen mit sulfonsäuregruppenhältigen Perfluorvinyläthern eingesetzt wird, wobei ein Molverhältnis von Arylamin zu Formaldehyd von 2,5 : 1 bis 15 : 1 angewendet wird.

Bei der Durchführung der Reaktion wird bevorzugt in zwei getrennten Reaktionsstufen gearbeitet. Zweckmäßig wird für die Kondensation eine Reaktionstemperatur im Bereich von 20 bis 90 °C und während der Umlagerung von 40 bis 190 °C gewählt, wobei vorteilhafterweise bei der Umlagerung eine höhere Temperatur angewendet wird als bei der Kondensation. Vorzugsweise wird die Kondensation jedoch bei 30 bis 50 °C und die Umlagerung bei 40 bis 190 °C durchgeführt.

Dabei werden besonders günstige Ergebnisse erhalten, wenn für die Umlagerung nicht gleich eine Temperatur im oberen Bereich der erfindungsgemäßen Temperaturspanne gewählt wird, sondern die Temperatur innerhalb dieses Bereiches stufenweise erhöht wird.

Der Katalysator kann hierbei gleich bei der Kondensation zugesetzt werden, es ist aber auch ebenso möglich, den Katalysator erst vor der Umlagerung dem Reaktionsgemisch beizufügen.

Der Formaldehyd wird vorzugsweise als wäßrige Lösung für die Reaktion eingesetzt, er kann aber auch gasförmig zugesetzt werden. Als formaldehydabgebende Substanz ist vor allem Paraformaldehyd zu nennen.

Das Wasser, das in die Kondensationsstufe eingeführt wird bzw. in dieser entsteht, kann vor Beginn der Umlagerung zur Hauptsache entfernt werden, z. B. durch Phasentrennung, wobei eine Restmenge an

Wasser im Reaktionsgemisch verbleibt. Es ist aber ebenso gut möglich, das Wasser im Reaktionsgemisch zu belassen, eine Verfahrensweise, die ein Arbeiten in einer einzigen Stufe ermöglicht. Auf das Ergebnis der Umsetzung bzw. Umlagerung hat die Menge des Wassers, die zugegen ist, praktisch keinen Einfluß, was zeigt, daß in Gegenwart der erfindungsgemäß gewählten Katalysators andere Verhältnisse herrschen als bei den früher verwendeten sauren Ionentauscherharzen.

Als Ausgangsprodukte sind eine Vielzahl von Arylaminen geeignet (siehe z. B. die europäische Patentanmeldung 0014927). Besonders geeignet sind jedoch Anilin, o-Toluidin, o-Chloranilin, o-Anisidin und N-Methylanilin.

Die Umsetzung kann diskontinuierlich oder kontinuierlich durchgeführt werden, wobei im letzteren Fall die in der ersten Stufe des Reaktionsablaufes gebildete Mischung der Kondensationsprodukte (N-Methylendiamine und Aminobenzylaniline) über ein Bett des erfindungsgemäß als Katalysator verwendeten Ionentauschers geleitet werden.

Bei batchweisem Arbeiten kann der Ionenaustauscher gleich zu Beginn der Umsetzung zugegeben werden. Es kann aber auch zuerst die Kondensationsreaktion ablaufen und anschließend der Ionenaustauscher zugegeben werden, der dann die Umlagerung zu den Endprodukten bewirkt und bei der Aufarbeitung wieder abgetrennt werden muß. Die Reaktionstemperaturen sowohl für die Kondensation als auch für die nachfolgende Umlagerung zu den Endprodukten beeinflussen in Abhängigkeit von der Reaktionsfähigkeit des eingesetzten Arylamins die Reaktionsgeschwindigkeit der gesamten Umsetzung und den Reinheitsgrad des jeweiligen Endproduktes, wie den Durchführungsbeispielen zu entnehmen ist. Ebenso ist das gewählte Molverhältnis von Arylamin zu Formaldehyd von Einfluß.

So kann z. B. bei einem Molverhältnis Anilin : Formaldehyd von 10 : 1 ein besonders hoher Anteil an zweikernigen Verbindungen (95,8 %) bei relativ niedrigen Umlagerungstemperaturen (85 °C) erhalten werden (siehe Beispiel 1). Würde in diesem speziellen Fall hingegen das Molverhältnis Anilin : Formaldehyd bei annähernd gleicher Umlagerungstemperatur auf 2,5 : 1 gesenkt, würde der Anteil an zweikernigen Verbindungen auf etwa 90 % absinken.

Weiters ist es möglich, durch Variation der Umlagerungstemperatur die prozentuelle Zusammensetzung innerhalb der zweikernigen Amine zu verändern. Werden beispielsweise bei kontinuierlichem Arbeiten in einem ionenaustauschergefüllten Rohrreaktor sämmtliche Temperaturzonen auf 130 °C gehalten, so kann bei einem Molverhältnis Anilin : Formaldehyd von 10 : 1 der 2,4-Diaminodiphenylmethananteil auf 23 % gesteigert werden, während der Anteil an 4,4'-Diaminodiphenylmethan entsprechend absinkt (siehe Beispiel 1 bzw. Beispiel 4).

Will man unter anderem bei Verwendung von Anilin einen besonders hohen Anteil an 4,4'-Diaminodiphenylmethan im Reaktionsgemisch erhalten, ist dies am besten zu bewerkstelligen, wenn die Umlagerungstemperaturen stufenweise erhöht werden (siehe Beispiel 5).

Die Reaktionszeiten sind temperaturabhängig und betragen wie üblich für die Kondensation 1 bis 4 Stunden und für die Umlagerung ca. 1 bis 7 Stunden.

Das Molverhältnis Arylamin zu Formaldehyd liegt zwischen 2,5 : 1 bis 15 : 1, wobei ein hohes Arylamin : Formaldehydverhältnis anstelle eines inerten Lösungsmittels, wie Xylol, günstigerweise auch dann\eingesetzt wird, wenn die Viskosität in unerwünschter Weise ansteigen würde.

Die erfindungsgemäß als Katalysator eingesetzten Copolymeren von Tetrafluoräthylen mit sulfonsäuregruppenhältigen Perfluorvinyläthern sind bekannt. Sie enthalten die Gruppierungen

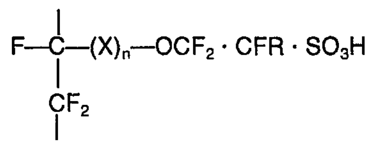

oder

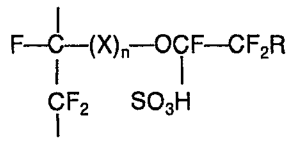

in denen n eine ganze Zahl von 1 bis 5, R ein Fluoratom oder ein einwertiges Perfluoralkylradikal und X die Gruppen $O(CF_2)_m$, $OCF_2CFY$ oder $OCFYCF_2$ bedeutet, wo m eine ganze Zahl von 2 bis 10 und Y ein Fluoratom oder eine Trifluormethylgruppe darstellt.

Die Herstellung derartiger Harze ist in den US-PSen 3 282 875 und 3 882 093 beschrieben. Unter diesen Katalysatoren sind solche besonders bevorzugt, die die Strukturformel (I)

(Siehe Formel Seite 4 f.).

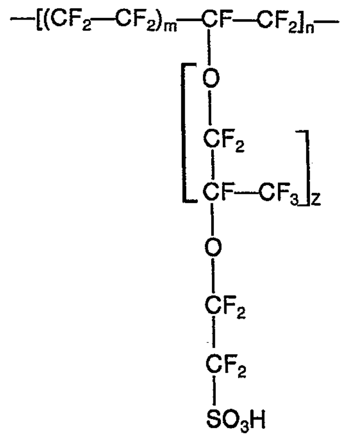

in welcher Formel m = 5 bis 13,5, n ungefähr 1 000 und Z 1, 2, 3 ... bedeutet, besitzen. Derartige Produkte sind unter dem Namen NAFION(R) im Handel.

Als Katalysator für das erfindungsgemäße Verfahren besitzen sie den Vorteil, daß sie über lange Produktionsperioden ihre Aktivität voll beibehalten. Sollten sei einmal an Aktivität verlieren, lassen sie sich durch Behandlung mit starken Mineralsäuren, auch mit konzentrierter Salpetersäure regenerieren.

Während der Umlagerung soll für einen möglichst innigen Kontakt zwischen Katalysator und der Mischung der Ausgangs- bzw. Vorkondensationsprodukte gesorgt werden.

Die erfindungsgemäß hergestellten Verbindungen werden mit Phosgen zu Isocyanaten umgesetzt und als Ausgangsprodukte für die Herstellung von Polyurethan-Hart- und Weichschäumen verwendet.

Die nachstehenden Beispiele sollen das erfindungsgemäße Verfahren näher erläutern.

## Beispiel 1

1. Stufe :

Herstellung der Kondensationsprodukte

In eine Lösung, bestehend aus 65,85 Mol Anilin und 3,0 kg Wasser, werden 6,58 Mol Formaldehyd als 37 %ige wäßrige Lösung (Molverhältnis Anilin : Formaldehyd = 10 : 1) unter intensivem Rühren bei Raumtemperatur eingetragen. Das Reaktionsgemisch wird anschließend 2 Stunden auf 50 °C gehalten und abgekühlt. Nach Abtrennen der wäßrigen Phase von der organischen Phase wird letztere direkt der säurekatalysierten Umlagerung zugeführt.

2. Stufe :

Umlagerung der in der ersten Stufe hergestellten Kondensationsprodukte zu den Endprodukten.

In einen Rohrreaktor (29,5 × 900 mm), der in 9 Temperaturzonen unterteilt ist, werden 520 g eines Copolymeren der Strukturformel I (NAFION(R)) einer Austauschkapazität von 0,83 meq/g in der H-Form als Katalysator eingefüllt. Anschließend werden dem Reaktor kontinuierlich 40 g/h der in der ersten Stufe hergestellten Kondensationsprodukte über eine Dosierpumpe zugeführt. Alle 9 Temperaturzonen werden auf 85 °C gehalten. Das am oberen Ende austretende Reaktionsgemisch weist nach Abdestillieren des überschüssigen Anilins folgende Zusammensetzung auf :

0,8 Gew.% 2,2'-Diaminodiphenylmethan
10,7 Gew.% 2,4'-Diaminodiphenylmethan
84,3 Gew.% 4,4'-Diaminodiphenylmethan und
3,6 Gew.% 3-Kernverbindung.

Der Rest von 0,6 Gew.% sind sowohl hier als auch in den folgenden Beispielen nicht näher identifizierte, mehrkernige Verbindungen.

## Beispiel 2

Beispiel 1 wird wiederholt mit dem einzigen Unterschied, daß alle 9 Temperaturzonen des Reaktors auf 100 °C gehalten werden.

Das erhaltene Polyamingemisch weist folgende Zusammensetzung auf :

1,7 Gew.% 2,2'-Diaminodiphenylmethan
18,1 Gew.% 2,4'-Diaminodiphenylmethan
76,0 Gew.% 4,4'-Diaminodiphenylmethan und
3,6 Gew.% 3-Kernverbindung

Beispiel 3

Herstellung der Vorkondensationsprodukte

In eine Lösung, bestehend aus 45 Mol Anilin und 2 kg Wasser, werden 15 Mol Formaldehyd (37 %ige Lösung, Molverhältnis Anilin : Formaldehyd 3 : 1) unter intensivem Rühren bei Raumtemperatur eingetragen. Das Reaktionsgemisch wird ca. 2 Stunden auf 50 °C gehalten und abgekühlt. Nach Abtrennen der wäßrigen Phase wird das Reaktionsgemisch direkt der Umlagerung mittels Ionenaustauscher zugeführt.

Nun wird mit dem so hergestellten Vorkondensationsprodukt gemäß Beispiel 1 vorgegangen, wobei in insgesamt 9 Zonen die Reaktionstemperaturen eingestellt werden wie folgt :

Zone 1 und 2 : 70 °C
Zone 3 und 4 : 80 °C
Zone 5 bis 8 : 90 °C
Zone 9 : 110 °C

Man erhält ein Polyamingemisch folgender Zusammensetzung :

1,5 Gew.% 2,2'-Diaminodiphenylmethan
19,2 Gew.% 2,4'-Diaminodiphenylmethan
71,8 Gew.% 4,4'-Diaminodiphenylmethan und
6,9 Gew.% 3- und höherkernige Arylamine

Beispiel 4

Beispiel 1 wird wiederholt mit dem einzigen Unterschied, daß alle 9 Temperaturzonen des Reaktors auf 130 °C gehalten werden.

Man erhält ein Polyamingemisch folgender Zusammensetzung :

2,7 Gew.% 2,2'-Diaminodiphenylmethan
23,2 Gew.% 2,4'-Diaminodiphenylmethan
69,3 Gew.% 4,4'-Diaminodiphenylmethan und
4,7 Gew.% 3-Kernverbindung

Beispiel 5

Beispiel 1 wird wiederholt mit dem einzigen Unterschied, daß der Rohrreaktor in verschiedene Temperaturzonen unterteilt wurde (Zone 1 + 2 70 °C ; Zone 3 bis 8 90 °C ; Zone 9 110 °C).

Das erhaltene Polyamingemisch weist folgende Zusammensetzung auf :

0,9 Gew.% 2,2'-Diaminodiphenylmethan
11,1 Gew.% 2,4'-Diaminodiphenylmethan
85,1 Gew.% 4,4'-Diaminodiphenylmethan und
2,8 Gew.% 3-Kernverbindung

Beispiel 6

Herstellung des Vorkondensates und des Endproduktes in einer Stufe

In ein Gemisch, bestehend aus 10 Mol o-Toluidin, 600 g des in Beispiel 1 angegebenen Katalysators in der $H^+$-Form und 2 kg Wasser, wird 1 Mol Formaldehyd als 37 %ige wäßrige Lösung (Molverhältnis Amin : Formaldehyd = 10 : 1) unter Rühren bei 90 °C innerhalb 1 Stunde eingetragen.

Das Gemisch wird noch 3,5 Stunden auf 90 °C gehalten. Nach Abfiltrieren des Katalysators werden das Wasser und das überschüssige o-Toluidin abdestilliert. Das Reaktionsgemisch enthält 93 Gew.% 4,4'-Diamino-3,3'-dimethyl-diphenylmethan. Die Verunreinigungen durch mehrkernige Verbindungen betragen 1 bis 2 Gew.%.

Beispiel 7

In eine Lösung, bestehend aus 10 Mol N-Methyl-anilin und 10 kg Wasser, wird 1 Mol Formaldehyd als

37 %ige wäßrige Lösung (Molverhältnis N-Methylanilin : Formaldehyd = 10 : 1) bei 20 °C unter intensivem Rühren innerhalb 30 min eingetragen.

Nach Abtrennen der wäßrigen Phase werden zur organischen Phase 400 g des Katalysators gemäß Beispiel 1 in der $H^+$-Form gegeben. Anschließend wird das Reaktionsgemisch 2 Stunden auf 120 °C gehalten. Der Katalysator wird abfiltriert und das überschüssige N-Methyl-anilin abdestilliert. Das Reaktionsgemisch enthält 94 Gew.% 4,4'-Diimethylamino-diphenylmethan. Verunreinigungen durch mehrkernige Verbindungen ca. 2 Gew.%.

Beispiel 8

In eine Lösung, bestehend aus 10 Mol o-Chlor-anilin und 2 kg Wasser, wird 1 Mol Formaldehyd als 37 %ige wäßrige Lösung (Molverhältnis o-Chlor-anilin : Formaldehyd = 10 : 1) bei 20 °C unter intensivem Rühren zugetropft.

Nach Abtrennen der wäßrigen Phase werden 500 g des Katalysators gemäß Beispiel 1 zugegeben und das Gemisch auf 130 °C aufgeheizt. Nach 2,5 Stunden wird der Katalysator abfiltriert und das überschüssige o-Chlor-anilin abdestilliert. Das Reaktionsgemisch enthält

83 Gew.% 4,4'-Diamino-3,3'-dichlor-diphenylmethan und
ca. 10 Gew.% 2,4'-Diamino-3,3'-dichlor-diphenylmethan.

Beispiel 9

In eine Lösung, bestehend aus 10 Mol 2-Methoxy-anilin und 4 kg Wasser, wird 1 Mol Formaldehyd als 37 %ige wäßrige Lösung (Molverhältnis 2-Methoxy-anilin : Formaldehyd = 10 : 1) unter intensivem Rühren eingetragen.

Nach Abtrennen der wäßrigen Phase werden zum Reaktionsgemisch 500 g des Katalysators gemäß Beispiel 1 zugegeben. Man hält das Reaktionsgemisch 1,5 Stunden auf 90 °C. Anschließend wird der Katalysator filtriert und das überschüssige 2-Methoxy-anilin abdestilliert. Das Reaktionsgemisch enthält

90 Gew.% 4,4'-Diamino-3,3'-Dimethoxy-diphenylmethan,
ca. 4 Gew.% 2,4'-Diamino-3,3'-dimethoxy-diphenylmethan und
ca. 3 Gew.% höhere Verunreinigungen.

**Ansprüche**

1. Verfahren zur Herstellung von Diaminen der Diphenylmethanreihe durch Kondensation von einkernigen Arylaminen mit freier para-Stellung gegenüber der Aminogruppe mit Formaldehyd oder unter Reaktionsbedingungen Formaldehyd abgebenden Substanzen und anschließende Umlagerung bei erhöhter Temperatur und in Gegenwart eines sauren Sulfonsäuregruppen enthaltenden Ionentauschers als Katalysator, wobei in beiden Stufen Wasser zugegen ist, dadurch gekennzeichnet, daß als Katalysator, der zumindest bei der Umlagerung zugegen ist, ein Copolymeres von Tetrafluoräthylen mit sulfonsäuregruppenhältigen Perfluorvinyläthern eingesetzt wird, wobei ein Molverhältnis von Arylamin : Formaldehyd von 2,5 : 1 bis 15 : 1 angewendet wird.

2. Verfahren nach Anspruch 1, dadurch gekennzeichnet, daß während der Kondensation eine Temperatur von 20 bis 90 °C und während der Umlagerung eine solche von 40 bis 190 °C angewendet wird.

3. Verfahren nach Anspruch 2, dadurch gekennzeichnet, daß die Temperatur während der Umlagerung innerhalb des Bereiches von 40 bis 190 °C stufenweise gesteigert wird.

4. Verfahren nach den Ansprüchen 1 bis 3, dadurch gekennzeichnet, daß die Kondensation bei einer Temperatur von 30 bis 50 °C durchgeführt wird.

5. Verfahren nach den Ansprüchen 1 bis 4, dadurch gekennzeichnet, daß die Umlagerung bei einer Temperatur von 80 bis 130 °C durchgeführt und gegebenenfalls innerhalb dieses Bereiches stufenweise gesteigert wird.

6. Verfahren nach den Ansprüchen 1 bis 5, dadurch gekennzeichnet, daß nach der Kondensation die Hauptmenge des Wassers abgetrennt wird.

7. Verfahren nach den Ansprüchen 1 und 6, dadurch gekennzeichnet, daß Kondensation und Umlagerung in einer einzigen Reaktionsstufe durchgeführt werden.

8. Verfahren nach den Ansprüchen 1 bis 7, dadurch gekennzeichnet, daß das Arylamin Anilin ist.

**Claims**

1. Process for the preparation of diamines of the diphenylmethane series by condensing mononuclear arylamines in which the para-position relative to the amino group is free, with formaldehyde or with substances which release formaldehyde under the reaction conditions, followed by rearrangement at elevated temperature and in the presence of an acid ion exchanger containing sulphonic acid groups, as

a catalyst, water being present in both steps, characterised in that a copolymer of tetrafluoroethylene with perfluorovinyl ethers containing sulphonic acid groups is used as the catalyst, which is present at least during the rearrangement, a molar ratio of arylamine : formaldehyde of 2.5 : 1 to 15 : 1 being used.

2. Process according to Claim 1, characterised in that a temperature of 20 to 90 °C is used during the condensation and a temperature of 40 to 190 °C is used during the rearrangement.

3. Process according to Claim 2, characterised in that the temperature during the rearrangement is increased stepwise within the range from 40 to 190 °C.

4. Process according to Claims 1 to 3, characterised in that the condensation is carried out at a temperature of 30 to 50 °C.

5. Process according to Claims 1 to 4, characterised in that the rearrangement is carried out at a temperature of 80 to 130 °C and, if appropriate, is increased stepwise within this range.

6. Process according to Claims 1 to 5, characterised in that the bulk of the water is separated off after the condensation.

7. Process according to Claims 1 and 6, characterised in that the condensation and rearrangement are carried out in a single reaction step.

8. Process according to Claims 1 to 7, characterised in that the arylamine is aniline.

**Revendications**

1. Procédé pour la préparation de diamines de la série du diphénylméthane par condensation d'arylamines monocycliques comportant une position para libre relativement au groupe amino avec du formaldéhyde ou des substances libérant du formaldéhyde dans les conditions de la réaction puis transposition à température élevée et en présence d'un échangeur d'ions acide contenant des groupes acide sulfonique à titre de catalyseur, de l'eau étant présente au cours des deux stades, caractérisé en ce qu'on utilise, comme catalyseur qui est présent au moins lors de la transposition, un copolymère de tétrafluoréthylène avec des éthers perfluorovinyliques contenant des groupes acide sulfonique, le rapport molaire entre l'arylamine et le formaldéhyde allant de 2,5 : 1 à 15 : 1.

2. Procédé suivant la revendication 1, caractérisé en ce qu'on utilise, pendant la condensation, une température de 20 à 90 °C, et pendant la transposition une température de 40 à 190 °C.

3. Procédé suivant la revendication 2, caractérisé en ce que la température est, pendant la transposition, augmentée par paliers à l'intérieur d'une gamme allant de 40 à 190 °C.

4. Procédé suivant l'une quelconque des revendications 1 à 3, caractérisé en ce que la condensation est effectuée à une température de 30 à 50 °C.

5. Procédé suivant l'une quelconque des revendications 1 à 4, caractérisé en ce que la transposition est effectuée à une température de 80 à 130 °C, qui est élevée par paliers le cas échéant à l'intérieur de cette gamme.

6. Procédé suivant l'une quelconque des revendications 1 à 5, caractérisé en ce que la plus grande partie de l'eau est séparée après la condensation.

7. Procédé suivant l'une quelconque des revendications 1 à 6, caractérisé en ce qu'on effectue la condensation et la transposition au cours d'un seul stade réactionnel.

8. Procédé suivant l'une quelconque des revendications 1 à 7, caractérisé en ce que l'arylamine est constituée par de l'aniline.